(19) Europäisches Patentamt
European Patent Office
Office européen des brevets

(11) Publication number: **0 399 422 A1**

# (12) EUROPEAN PATENT APPLICATION

(21) Application number: **90109570.3**

(22) Date of filing: **19.05.90**

(51) Int. Cl.5: **C07C 275/30, C07C 323/44, C07C 275/26, C07D 333/12, C07D 213/36, C07D 311/20, C07D 335/06, A61K 31/17**

(30) Priority: **25.05.89 JP 134321/89**
**17.01.90 JP 9264/90**

(43) Date of publication of application:
**28.11.90 Bulletin 90/48**

(84) Designated Contracting States:
**AT BE CH DE DK ES FR GB GR IT LI LU NL SE**

(71) Applicant: **TAKEDA CHEMICAL INDUSTRIES, LTD.**
**3-6, Doshomachi 2-chome Chuo-ku**
**Osaka 541(JP)**

(72) Inventor: **Tawada, Hiroyuki**
**11-1 Miyanokawahaea 1-chome**
**Takatsuki, Osaka 569(US)**
Inventor: **Meguro, Kanji**
**2-21 Mondosh**
**Nishinomiya, Hyogo 662(JP)**
Inventor: **Ikeda, Hitoshi**
**13-712, 3 Nishi-iwata 3-chome**
**Higashiosaka, Osaka 578(JP)**

(74) Representative: **von Kreisler, Alek, Dipl.-Chem. et al**
**Patentanwälte Von Kreisler-Selting-Werner,**
**Deichmannhaus am Hauptbahnhof**
**D-5000 Köln 1(DE)**

## (54) Benzocycloalkane derivatives and production thereof.

(57) Benzocycloalkane derivative of the formula

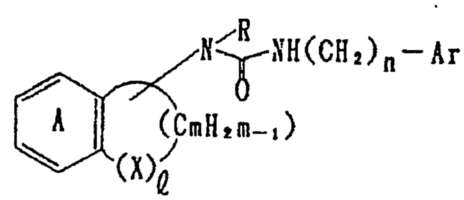

wherein R is a hydrogen atom or a hydrocarbon residue, which may optionally have one or more substituents, Ar is an aromatic group, X is an oxygen or sulfur atom, ℓ is 0 or 1, m is 3 to 6 and n is 0 to 2, wherein the ring A and the group Ar each may optionally have one or more substituents, with the proviso that compounds are excluded, wherein Ar is

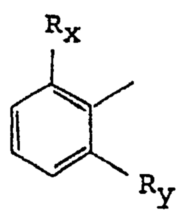

EP 0 399 422 A1

whereby $R_x$ and $R_y$ are independently selected from alkyl or alkoxy of from one to seven carbon atoms, or its pharmaceutically acceptable salt, which is useful as inhibitors for acyl-CoA : cholesterolacyltransferase.

# BENZOCYCLOALKANE DERIVATIVES AND PRODUCTION THEREOF

## BACKGROUND OF THE INVENTION

### 1. Field of the invention

This invention relates to novel benzocycloalkane derivatives having potent acyl-CoA:cholesterol acyl transferase (ACAT) inhibiting activity.

The compounds according to the invention inhibit, in mammals, the absorption of cholesterol through the intestinal tract and the accumulation of cholesterol esters on the arterial wall and therefore are useful as prophylactic and therapeutic agents for hypercholesterolemia, atherosclerosis and various diseases resulting from these (e.g. ischemic cardic diseases such as myocardial infarction, cerebrovascular disturbance such as cerebral infarction and cerebral apoplexy, etc.).

### 2. Description of the prior art

Urea derivatives having ACAT inhibiting activity are disclosed in Japanese Patent Application KOKAI Nos. 316761/1988 and 93569/1989 and U.S. Patent No. 4,623,662. However, any urea derivatives having a benzocycloalkyl substituent directly on a urea nitrogen atom have not been synthesized as yet.

No urea compounds having a benzocycloalkyl group as a direct substituent on a urea nitrogen atom have been known to have good ACAT inhibiting and blood cholesterol lowering activities or, in other words, be useful as agents for atherosclerosis.

The present inventors synthesized various novel urea derivatives having a benzocycloalkyl group as a direct substituent on a urea nitrogen atom and intensively investigated their activities and, as a result, found that novel compounds have excellent ACAT inhibiting activity and are useful as a drug for atherosclerosis.

## SUMMARY OF THE INVENTION

This invention relates to

(1) A novel benzocycloalkane derivative of the formula (I):

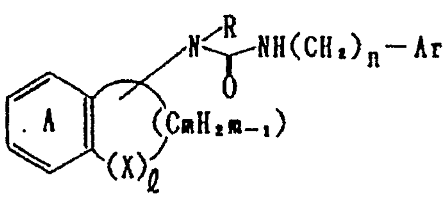

(I)

wherein R is H or a hydrocarbon residue, which may optionally have one or more substituents, Ar is an aromatic group, X is an oxygen or sulfur atom, ℓ is 0 or 1, m is 3 to 6 and n is 0 to 2, wherein the ring A and the group Ar each may optionally have one or more substituents, with the proviso that compounds are excluded, wherein Ar is

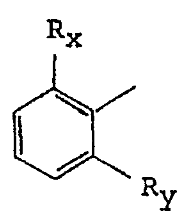

whereby $R_x$ and $R_y$ are independently selected from alkyl or alkoxy of from one to seven carbon atoms, or its pharmaceutically acceptable salt, have excellent ACAT inhibiting activity;

(2) A method of producing the novel benzocycloalkane derivative of formula (I); and

(3) An ACAT inhibitor composition which contains any of the compound of formula (I).

## DESCRIPTION OF THE PREFERRD EMBODIMENT

Referring to the formula (I), R is H or a hydrocarbon residue, which may optionally have one or more substituents. The hydrocarbon residue represented by R is, for example, an alkyl, aryl or aralkyl group. The alkyl group represented by R is preferably a straight, branched or cyclic one containing 1 to 8 carbon atoms, such as methyl, ethyl, propyl, isopropyl, cyclopropyl, butyl, isobutyl, sec-butyl, tert-butyl, cyclopropylmethyl, pentyl, isopentyl, neopentyl, cyclopentyl, hexyl, cyclohexyl, heptyl, cyclohexylmethyl or octyl. The aryl group represented by R is preferably an aryl group containing 6 to 10 carbon atoms, such as phenyl or naphthyl. The aralkyl group represented by R preferably contains 7 to 16 carbon atoms and includes, among others, benzyl, 1-phenylethyl, 2-phenylethyl, 1-phenylpropyl, 2-phenylpropyl, 3-phenylpropyl, diphenylmethyl, o-, m- or p-methylbenzyl, o-, m- or p-ethylbenzyl, o-, m- or p-isopropylbenzyl, o-, m- or p-tert-butylbenzyl, 2,3-, 2,4-, 2,5-, 2,6-, 3,4- or 3,5-dimethylbenzyl, 2,3,4-, 3,4,5- or 2,4,6-trimethylbenzyl, 5-isopropyl-2-methylbenzyl, 2-isopropyl-5-methylbenzyl, 2-methyl-5-tert-butylbenzyl, 2,4-, 2,5- or 3,5-diisopropylbenzyl, 3,5-di-tert-butylbenzyl, 1-(2-methylphenyl)ethyl, 1-(3-metylphenyl)ethyl, 1-(4-methylphenyl)ethyl, 1-(2-isopropylphenyl)ethyl, 1-(3-isopropylphenyl)ethyl, 1-(4-isopropylphenyl)ethyl, 1-(2-tert-butylphenyl)ethyl, 1-(4-tert-butylphenyl)ethyl, 1-(2-isopropyl-4-methylphenyl)ethyl, 1-(4-isopropyl-2-methylphenyl)ethyl, 1-(2,4-dimethylphenyl)ethyl, 1-(2,5-dimethylphenyl)ethyl, 1-(3,5-dimethylphenyl)ethyl and 1-(3,5-di-tert-butylphenyl)ethyl. These hydrocarbon residues represented by R may have 1 to 5 substituents, which may be the same or different. As such substituents, there may be mentioned halogen atoms, lower alkoxy, which may optionally be halogenated, lower alkylthio, which may optionally be halogenated, nitro, carboxy, esterified carboxy, hydroxy, $C_{1-3}$ acyl (e.g. formyl, acetyl, propionyl) and heterocyclic groups. Examples of the halogen atoms as such substituents are fluorine, chlorine, bromine and iodine. The lower alkoxy and lower alkylthio groups, which may optionally be halogenated, are, for example, those lower alkoxy and lower alkylthio groups which are derived from straight or branched lower alkyl groups containing 1 to 6 carbon atoms (e.g. methyl, ethyl, propyl, isopropyl, butyl, isobutyl, sec-butyl, tert-butyl, pentyl, isopentyl, hexyl) by binding with an oxygen or sulfur atom, respectively, and may optionally be halogenated (fluorinated, chlorinated, brominated or iodinated). The esterified carboxy is, for example, a $C_{1-6}$ alkyl-esterified carboxy group, such as methyl-, ethyl-, propyl-, isopropyl-, butyl-, isobutyl-, sec-butyl-, tert-butyl-, pentyl- or hexyl-esterified carboxy. The heterocyclic groups are, for example, 5- or 6-membered aromatic heterocyclic groups, preferably those containing at least one heteroatom selected from N, S and O, such as 2- or 3-thienyl, 2- or 3-furyl, and 2-, 3-, or 4-pyridyl. Preferred examples of R are benzyl and substituted benzyl groups, in particular substituted benzyl groups having 1 to 4 substituents each selected from among $C_{1-4}$ alkyl groups (e.g. metyl, ethyl, propyl, isopropyl, butyl, isobutyl, sec-butyl, tert-butyl), $C_{1-4}$ alkoxy groups, hydroxy, halogens and so forth. When R is a hydrocarbon residue (in particular benzyl or the like) substituted by a hydroxy group, the compounds (I) additionally have antioxidant activity.

The aromatic group Ar may be isocyclic or heterocyclic. As examples of the aromatic isocyclic group, there may be mentioned those aryl groups mentioned hereinabove and, as examples of the aromatic heterocyclic group, those 5- or 6-membered aromatic heterocyclic groups mentioned hereinabove.

The ring A and the above-mentioned group Ar each may have 1 to 5 substituents, which may be the same or different, at any position or positions on the ring thereof. As the substituents on the ring A and/or the group Ar, there may be mentioned those substituents which the group R may optionally have as well as lower alkyl groups, which may optionally be halogenated, for example straight or branched lower alkyl groups containing 1 to 6 carbon atoms and groups derived therefrom by substitution with 2 to 5 halogen atoms, such as methyl, chloromethyl, difluoromethyl, trichloromethyl, trifluoromethyl, ethyl, 2-bromoethyl, 2,2,2-trifluoroethyl, pentafluoroethyl, propyl, 3,3,3-trifluoropropyl, isopropyl, 2-trifluoromethylethyl, butyl, 4,4,4-trifluorobutyl, isobutyl, sec-butyl, tert-butyl, pentyl, isopentyl, neopentyl, 5,5,5-trifluoropentyl, 4-trifluoromethylbutyl, hexyl, 6,6,6-trifluorohexyl, 5-trifluorohexyl and 5-trifluoromethylpentyl.

Preferred examples of Ar are mono- or di-halogen (e.g. F)-substituted phenyl groups. The ring A is preferably unsubstituted. n is preferably equal to 0. $\ell$ stands for 0 or 1 and m for an integer of 3 to 6.

Thus, the group

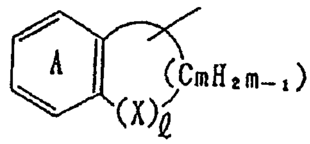

specifically includes, among others, the following:

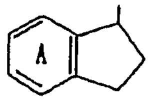 , 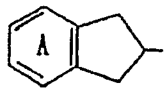 , 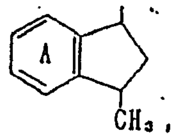 , 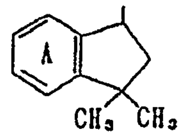 ,

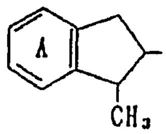 , 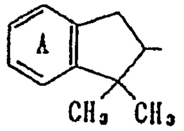 , 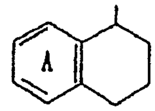 , 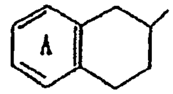 ,

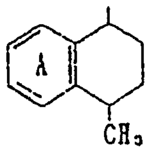 , 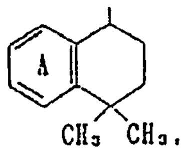 , 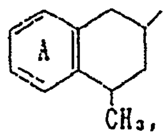 , 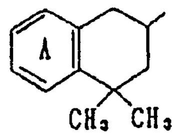 ,

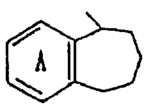 , 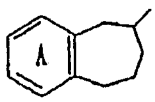 , 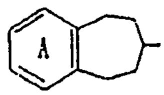 , 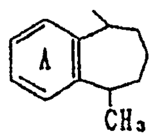 ,

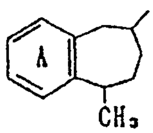 , 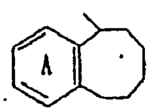 , 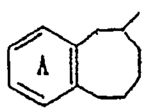 , 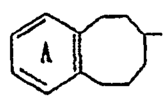 ,

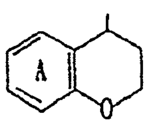 , 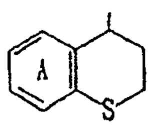 , 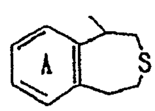 , 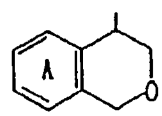 ,

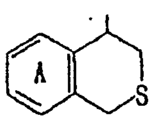 , 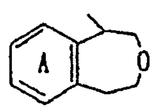 , etc.

The above compound (I) may be in the form of its pharmaceutically acceptable salt, e.g. an alkali metal salt with the carboxyl group of the compound (I), e.g. sodium salt or potassium salt.

The benzocycloalkane derivative of the formula (I) can be produced, for example, by reacting a compound of the formula (II):

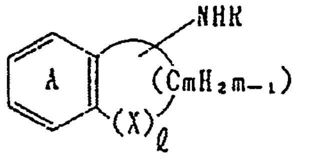

(II)

or a salt thereof with a compound of the formula (III)

$Ar-(CH_2)_nNCO$ (III)

In formulas (II) and (III), the symbols are as defined hereinabove.

The salt of the aminobenzocycloalkane derivative (II) includes, among others, salts with inorganic or organic acids, such as hydrochloric acid, hydrobromic acid, sulfuric acid, phosphoric acid, methanesulfonic acid, benzenesulfonic acid, p-toluenesulfonic acid, fumaric acid, maleic acid, citric acid and tartaric acid. This reaction is generally carried out in an appropriate solvent. Any solvent inert to the reaction can be used as the solvent. Thus, for example, use may be made of ethers, such as ethyl ether, isopropyl ether, dimethoxyethane, tetrahydrofuran and dioxane, aromatic hydrocarbons, such as benzene, toluene and xylene, esters, such as methyl acetate and ethyl acetate, ketones, such as acetone and methyl ethyl ketone, halogenated hydrocarbons, such as dichloromethane and chloroform, pyridine, N,N-dimethylformamide and dimethyl sulfoxide. In cases where (II) is submitted to the reaction in the form of a salt, the reaction will generally proceed significantly if carried out in the presence of a base (e.g. trimethylamine, triethylamine, pyridine, picoline, sodium methylate, sodium ethylate, sodium hydroxide, potassium hydroxide, sodium carbonate, potassium carbonate) for effecting desalting. Such base is used in an amount of 1 to 3 moles, preferably 1 to 1.5 moles, per mole of (II). The reaction is generally carried out at -10° C to 150° C, preferably 0° C to 80° C. (III) is used in an amount of about 1 to 5 equivalents, preferably 1 to 2 equivalents, relative to (II). The reaction period required may vary depending on the starting materials, solvent, reaction temperature and other factors. Generally, however, the reaction is carried out for a period of 5 minutes to 24 hours, preferably 10 minutes to 6 hours.

Among the compounds (I) thus produced, those in which the ring A, the group Ar and/or the group R has at least one lower alkoxy group as a substituent on the benzene ring thereof may be converted to the corresponding compounds having a hydroxyl group in place of said lower alkoxy group by reacting the compounds (I) either as they are in the respective reaction mixtures or after isolation thereof by known procedures, such as mentioned hereinbelow, with boron tribromide, for instance. This reaction is generally carried out in a solvent (e.g. halogenated hydrocarbon, such as dichloromethane, chloroform or carbon tetrachloride, aromatic hydrocarbon such as benzene or toluene) at about -20° C to 80° C, preferably -5° C to 30° C. Boron tribromide is used in an amount of about 1 to 10 equivalents, preferably about 1 to 5 equivalents, per lower alkoxy group.

The desired compounds (I) thus produced can be purified and recovered by using per se known separation or purification procedures (e.g. concentration, solvent extraction, column chromatography, recrystallization).

Preferred among the compounds (I) producible in the above manner are, for example, compounds of the formula ($I^a$):

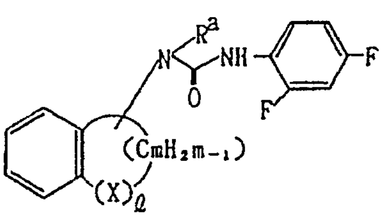

($I^a$)

wherein $R^a$ is a benzyl group, which may optionally have one or more substituents, X is an oxygen or sulfur atom, and ℓ and m are as defined above, and compounds of the formula ($I^b$):

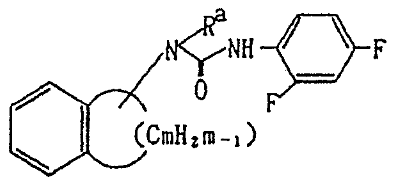

(I b)

wherein $R^a$ and m are as defined above. In the above formulas, the benzyl group represented by $R^a$ may have one or more of the same substituents as mentioned hereinabove for R.

The compounds (I) have potent acyl-CoA:cholesterol acyl transferase (ACAT) inhibiting activity as well as weak acute toxicity and weak chronic toxicity. ACAT is an enzyme catalyzing the esterification of cholesterol with higher fatty acids. It is known that ACAT plays an important role in the absorption of cholesterol in the small intestine and the intracellular accumulation of cholesterol esters. Therefore, an ACAT inhibitor can inhibit absorption of dietary cholesterol through the intestinal tract to prevent blood cholesterol level from increasing, and at the same time can inhibit intracellular cholesterol ester accumulation in arteriosclerotic foci to thereby impede the progress of atherosclerosis. Accordingly, the compounds (I) according to the invention are useful as safe prophylactic and therapeutic agents for hypercholesterolemia, atherosclerosis and diseases resulting from these (e.g. ischemic heart diseases such as myocardial infarction, cerebrovascular diseases such as cerebral infarction and cerebral apoplexy) in mammals (e.g. mouse, rat, hamster, rabbit, cat, dog, horse, cattle, sheep, monkey, human).

For use as the above-mentioned medicinals, the compound of formula (I) can be admixed with pharmacologically acceptable, appropriate carriers, excipients or diluents to give such dosage forms as powders, granules, tablets, capsules or injections, which are to be administered either orally or nonorally. For the purpose of inhibiting cholesterol absorption, the oral route of administration is preferred. The dose may vary depending on the kind of compound (I), route of administration, symptom, patient's age and other factors. In the case of oral administration to adult hypercholesterolemic patients, for instance, the daily dose per kilogram of body weight is within the range of about 0.005 to 100 mg, preferably about 0.05 to 50 mg, more preferably 0.5 to 10 mg, and such daily dose is preferably administered as a single dose or in two or three divided doses.

The starting compounds for the production of the compounds (I) according to the invention can be produced by a per se known method, for example, in the following manner:

[Process A]

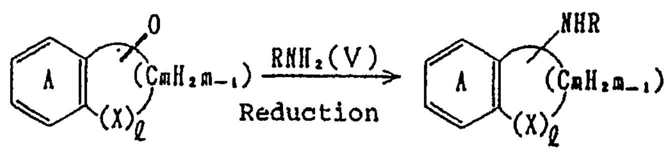


(IV) (II)

(The symbols in the above formulas are as defined hereinabove.)

[Process B]

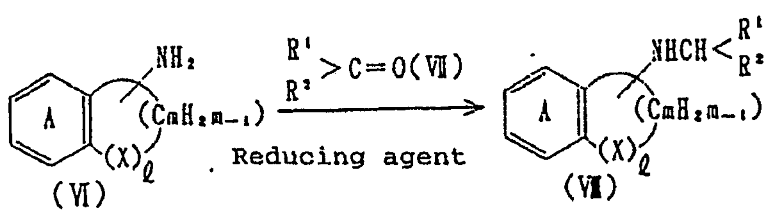


(VI) (VII)

(In the above formulas, $R^1$ and $R^2$ each is a hydrogen atom or a hydrocarbon group, which may optionally have one or more substituents, and the other symbols are as defined hereinabove.)

[Process C]

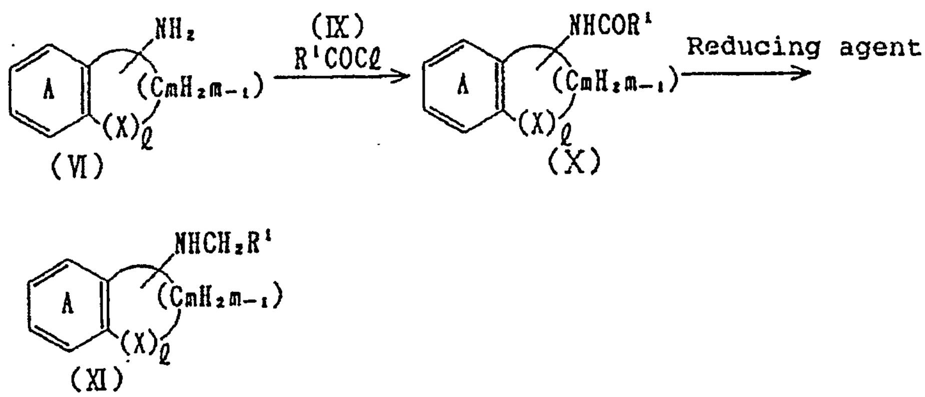


(VI) (X) (XI)

(The symbols in the above formulas are as defined hereinabove.)

[Process A]

The reductive alkylation of the amine (V) with the benzocycloalkanone (IV) can be generally carried out in a solvent. The solvent to be used may be any solvent, if it is inert to the reaction. Thus, for example, alcohols, such as methanol, ethanol and isopropanol, ethers, such as ethyl ether, isopropyl ether, dimethoxyethane, tetrahydrofuran and dioxane, aromatic hydrocarbons, such as benzene, toluene and xylene, dimethylformamide and dimethyl sulfoxide may be used. Mixed solvents composed of these may also be used. The reducing agent to be used in this process is, for example, sodium borohydride, lithium borohydride, sodium cyanoborohydride, lithium aluminum hydride or the like. Generally, such reducing agent is used in an amount of 0.5 to 5 moles, preferably 0.5 to 2 moles, per mole of (IV). Such reduction can be performed generally at -10° C to 150° C, preferably at -5° C to 100° C. The reaction period is generally 15 minutes to 24 hours, preferably 30 minutes to 8 hours.

This reduction reaction can be carried out not only by the use of the reducing agent mentioned above but also in the manner of catalytic reduction using an appropriate catalyst. As such catalyst, there may be mentioned, for example, palladium catalysts, such as palladium black, palladium on charcoal and palladium chloride, platinum catalysts, such as platinum oxide and platinum black, and Raney nickel. The catalyst is used generally in an amount of 0.001 to 2 moles, preferably in an amount of 0.01 to 1 mole, per mole of (IV). The reaction pressure is 1 to 100 atmospheres/$cm^2$, preferably 1 to 20 atmospheres/$cm^2$. Such reduction reaction can be conducted generally at -10° C to 150° C, preferably at -5° C to 100° C. The reaction period is generally 30 minutes to 12 hours, preferably 30 minutes to 5 hours.

The above-mentioned reduction reaction and catalytic reduction can be conducted in the presence of an acid catalyst for promoting the reaction. Useful as said acid catalyst are organic acids, such as formic acid, acetic acid, propionic acid, trifluoroacetic acid, methanesulfonic acid, benzenesulfonic acid, p-toluenesulfonic acid and camphorsulfonic acid, and inorganic acids, such as hydrochloric acid, sulfuric acid and phosphoric acid. Generally, the acid is used in an amount of 0.5 to 20 moles, preferably 1 to 10 moles, per mole of (IV).

[Process B]

The starting material (VIII) can be produced by reductive alkylation of the aminobenzocycloalkane (VI) with the carbonyl compound (VII) essentially in the same manner as the process A mentioned above.

[Process C]

The reaction (acylation) of the compound (VI) with the acid chloride (IX) is carried out generally in a solvent. Usable as such solvent are any solvents inert to the reaction, for example halogenated hydrocarbons, such as carbon tetrachloride, chloroform, dichloromethane and 1,1,2,2-tetrachloroethane, esters, such as ethyl acetate and methyl acetate, ketones, such as acetone and methyl ethyl ketone, ethers, such as ethyl ether, isopropyl ether, tetrahydrofuran and dioxane, aromatic hydrocarbons, such as benzene, toluene

and xylene, dimethylformamide and dimethyl sulfoxide as well as mixtures of such solvents. When the reaction is carried out in the manner of Schotten-Baumann acylation, water and mixtures of water and the solvents mentioned above can also be used. Reactive derivatives (e.g. mixed acid anhydrides, active esters) of the corresponding acid may be used in lieu of (IX). This acylation reaction may be conducted in the presence of a deacidifying agent or acid acceptor. As examples of such deacidifying agent, there may be mentioned organic bases, such as trimethylamine, triethylamine, pyridine, picoline, dimethylaniline and diethylaniline, and inorganic bases, such as sodium hydroxide, potassium hydroxide, sodium carbonate, potassium carbonate, sodium hydrogen carbonate and potassium hydrogen carbonate. Generally, the deacidifying agent is used in an amount of 1 to 5 moles, preferably 1 to 2 moles, per mole of (IX). The reaction temperature is generally -20 °C to 100 °C, preferably -10 °C to 50 °C. The reaction period is generally 15 minutes to 24 hours, preferably 30 minutes to 8 hours. The acylamino compound (X) thus obtained is reacted with a reducing agent to give (XI). As examples of said reducing agent, there may be mentioned those reducing agents mentioned hereinabove with reference to the process A as well as a mixture of a Lewis acid (e.g. aluminum chloride, zinc chloride, boron trifluoride etherate) and lithium aluminum hydride and a mixture of an organic acid (e.g. acetic acid, trifluoroacetic acid) and sodium borohydride, among others. Any solvent inert to the reaction may be used. As examples, there may be mentioned those solvents usable in the process A. The reaction is generally carried out at a temperature of -10 °C to 150 °C, preferably -5 °C to 100 °C, for a period of 30 minutes to 20 hours, preferably 30 minutes to 8 hours. The starting compound (II) as well as the compounds (VIII) and (XI) [each being a species of (II)] are used as the materials for the production of the compounds according to the invention, either after isolation thereof by known procedures such as mentioned above or in the form of reaction mixtures.

The starting compounds (IV) and (VI) can be synthesized by known processes, for example by the processes described by Miyake et al. in Journal of the Takeda Research Laboratories, 44, 171 (1985) and idem., 45, 122 (1985) or modifications thereof. The compounds (V), (VII) and (IX) can be synthesized by *per se* known processes.

## Activity

The following pharmacological test results indicate that the benzocycloalkane derivatives (I) according to the invention are of great utility.

### 1. Acyl-CoA:cholesterol acyl transferase (ACAT) inhibiting activity

[Test method]

An ACAT enzyme preparation was obtained from the small intestine mucosa microsome fraction from male Sprague-Dawley rats of 6 weeks of age fasted for 20 hours, as described by Heider et al. [Journal of Lipid Research, 24, 1127 (1982)].

The ACAT activity was calculated by determining the yield of a labeled cholesterol ester from [1-$^{14}$C]-oleoyl-CoA and endogenous cholesterol using the method of Helgerud et al. [Journal of Lipid Research, 22, 271 (1981)].

[Results]

The inhibition percentages of the labeled cholesterol ester formation at $10^{-6}$ M of the test compounds are shown in Table 1 as ACAT inhibiting activity indices.

Table 1

| Test compound (Example No.) | % ACAT inhibition | Test compound (Example No.) | % ACAT inhibition |
|---|---|---|---|
| 1 | 84 | 35 | 58 |
| 2 | 65 | 36 | 72 |
| 5 | 76 | 37 | 93 |
| 6 | 80 | 39 | 89 |
| 7 | 72 | 40 | 83 |
| 9 | 53 | 41 | 95 |
| 10 | 72 | 42 | 62 |
| 11 | 64 | 44 | 63 |
| 12 | 77 | 45 | 96 |
| 13 | 86 | 46 | 77 |
| 15 | 88 | 49 | 89 |
| 16 | 86 | 50 | 85 |
| 17 | 75 | 56 | 79 |
| 19 | 55 | 57 | 84 |
| 20 | 72 | 58 | 94 |
| 22 | 55 | 59 | 80 |
| 23 | 57 | 60 | 83 |
| 26 | 60 | 61 | 53 |
| 27 | 58 | 62 | 69 |
| 28 | 56 | 66 | 66 |
| 33 | 57 | 67 | 73 |

The data given in Table 1 prove that the compounds (I) according to the invention as represented by the compounds obtained in the respective examples have potent ACAT inhibiting activity.

2. Plasma cholesterol lowering activity in cholesterol-loaded rats

[Test method]

Male Sprague-Dawley rats of 7 weeks of age were fed with a 1% cholesterol diet (containing 0.5% cholic acid and 5% olive oil) for 3 days, then grouped by plasma cholesterol level and further fed with the same diet containing 0.01% test compound for 4 days. Blood samples were collected at 8:30 a.m. to 10:00 a.m. while the animals were satiated, and plasma cholesterol levels were determined enzymatically. Intakes of the compound were calculated from food intake data.

[Results]

As can be seen in Table 2, the test compound caused a significant decrease in plasma cholesterol level in cholesterol-loaded animals.

Table 2

| Test compound (Example No.) | Dose mg/kg/day | Plasma cholesterol mg/dl |
|---|---|---|
| Control group | 0 | 266 + 46 |
| 1 | 8.5 | 128 + 47* |

* $p<0.01$

The data given in Table 2 prove that the compounds (I) according to the invention as represented by the compound of Example 1 have potent plasma cholesterol lowering activity.

## Examples

The following Reference Examples and working Examples are further illustrative of the present invention. It is to be noted, however, that such Examples are by no means limitative of the scope of the invention.

In the Reference Examples and Examples, elution in column chromatography was performed under observation by thin layer chromatography (TLC). In the TLC observation, silica gel 60 $F_{245}$ (Merck) TLC plates were used, the developing solvent was the same as the solvent used as the eluent in column chromatography, and a UV detector was used as the means of detection. Silica gel 60 (Merck; 70-230 mesh) was used as the column packing silica gel.

In the Examples and Reference Examples, the following abbreviations are used:
mg for milligram(s); g for gram(s); mℓ for milliliter(s); mp for melting point.

The term "room temperature" means a temperature of about 15° C to about 25° C.

### Example 1

Triethylamine (0.28 ml) was added to a suspension of 2-(2-chlorobenzylamino)indan hydrochloride (0.59 g) in dichloromethane (6.0 ml), the mixture was stirred for 10 minutes and 2,4-difluorophenyl isocyanate (0.26 ml) was then added dropwise. The resulting mixture was stirred for 30 minutes, then washed with water and dried over anhydrous $MgSO_4$. The solvent was distilled off and the residue was crystallized by addition of ether-hexane to give N-(2-chlorobenzyl)-N′-(2,4-difluorophenyl)-N-(2-indanyl)urea (0.78 g, 94.0%). Recrystallization from ethanol gave colorless prisms (0.59 g, 71.7%).
mp 119-120° C.
Elemental analysis (for $C_{23}H_{19}C\ell F_2N_2O$)
Calculated: C, 66.91; H, 4.64; N, 6.79;
Found: C, 66.83; H, 4.66; N, 6.73.

The compounds of Examples 2 to 51 were obtained by reacting the corresponding aminobenzocycloalkane derivatives with the corresponding isocyanates in the same manner as above.

### Example 2

N-Benzyl-N′-(2,4-difluorophenyl)-N-(2- indanyl)urea:
mp 67-68° C (recrystallized from ethanol-hexane). Yield 94.0%.
Elemental analysis (for $C_{23}H_{20}F_2N_2O$)
Calculated: C, 73.00; H, 5.33; N, 7.40;
Found: C, 72.87; H, 5.30; N, 7.36.

### Example 3

N-(2,4-Difluorophenyl)-N′-(2-indanyl)-N′-phenylurea: mp 122-123°C (recrystallized from ethanol). Yield 91.7%.
Elemental analysis (for $C_{22}H_{18}F_2N_2O$)
Calculated: C, 72.52; H, 4.98; N, 7.69;
Found: C, 72.25; H, 4.98; N, 7.61.

Example 4

N-(2,4-Difluorophenyl)-N′-(2-indanyl)-N′-(2-phenylethyl)urea: mp 82-84°C (recrystallized from ethanol-ether). Yield 76.5%.
Elemental analysis (for $C_{24}H_{22}F_2N_2O$)
Calculated: C, 73.45; H, 5.65; N, 7.14;
Found: C, 73.09; H, 5.60; N, 6.80.

Example 5

N-(4-Chlorobenzyl)-N′-(2,4-difluorophenyl)-N-(2-indanyl)urea: mp 98-99°C (recrystallized from ethanol). Yield 88.0%.
Elemental analysis (for $C_{22}H_{19}C\ell F_2N_2O$)
Calculated: C, 66.91; H, 4.64; N, 6.79;
Found: C, 66.82; H, 4.63; N, 6.74.

Example 6

N-(2,4-Difluorophenyl)-N′-(2-indanyl)-N′-(1-phenylethyl)urea: mp 119-120°C (recrystallized from ethanol). Yield 90.6%.
Elemental analysis (for $C_{24}H_{22}F_2N_2O$)
Calculated: C, 73.45; H, 5.65; N, 7.14;
Found: C, 73.47; H, 5.67; N, 7.04.

Example 7

N-(2,4-Difluorophenyl)-N′-(2-indanyl)-N′-(1- phenylpropyl)urea: mp 118-119°C (recrystallized from ethanol). Yield 99.0%.
Elemental analysis (for $C_{25}H_{24}F_2N_2O$)
Calculated: C, 73.87; H, 5.95; N, 6.89;
Found: C, 74.06; H, 5.98; N, 6.82.

Example 8

N-(2,4-Difluorophenyl)-N′-(2-indanyl)-N′-(2-methyl-1-phenylpropyl)urea: mp 172-173°C (recrystallized from ethanol). Yield 96.4%.
Elemental analysis (for $C_{26}H_{26}F_2N_2O$)
Calculated: C, 74.27; H, 6.23; N, 6.66;
Found: C, 74.26; H, 6.27; N, 6.57.

Example 9

N-(2,4-Difluorophenyl)-N′-(2-indanyl)-N′-diphenyl methylurea: mp 141-142°C (recrystallized from ethanol). Yield 92.3%.
Elemental analysis (for $C_{29}H_{24}F_2N_2O$)
Calculated: C, 76.63; H, 5.32; N, 6.16;

Found: C, 76.64; H, 5.37; N, 6.01.

Example 10

N-(2,4-Difluorophenyl)-N′-(2-indanyl)-N′-(2-methylbenzyl)urea: mp 94-95 °C (recrystallized from isopropyl ether). Yield 57.5%.
Elemental analysis (for $C_{24}H_{22}F_2N_2O$)
Calculated: C, 73.45; H, 5.65; N, 7.14;
Found: C, 73.50; H, 5.70; N, 7.09.

Example 11

N-(2,4-Difluorophenyl)-N′-(2-indanyl)-N′-(3-methylbenzyl)urea: mp 72-73 °C (recrystallized from isopropyl ether-hexane). Yield 91.0%.
Elemental analysis (for $C_{24}H_{22}F_2N_2O$)
Calculated: C, 73.45; H, 5.65; N, 7.14;
Found: C, 73.46; H, 5.65; N, 7.10.

Example 12

N-(2,4-Difluorophenyl)-N′-(2-indanyl)-N′-(4-methylbenzyl)urea: mp 91-92 °C (recrystallized from isopropyl ether). Yield 76.9%.
Elemental analysis (for $C_{24}H_{22}F_2N_2O$)
Calculated: C, 73.45; H, 5.65; N, 7.14;
Found: C, 73.68; H, 5.62; N, 7.14.

Example 13

N-(4-Isopropylbenzyl)-N′-(2,4-difluorophenyl)-N-(2-indanyl)urea: mp 119-120 °C (recrystallized from isopropyl ether). Yield 97.6%. Elemental analysis (for $C_{26}H_{26}F_2N_2O$)
Calculated: C, 74.27; H, 6.23; N, 6.66;
Found: C, 74.21; H, 6.22; N, 6.59.

Example 14

N-(4-tert-Butylbenzyl)-N′-(2,4-difluorophenyl)-N-(2-indanyl)urea: mp 114-115 °C (recrystallized from ethanol). Yield 94.3%.
Elemental analysis (for $C_{27}H_{28}F_2N_2O$)
Calculated: C, 74.63; H, 6.49; N, 6.45;
Found: C, 74.96; H, 6.54; N, 6.38.

Example 15

N-(2,4-Difluorophenyl)-N′-(2-indanyl)-N′-(2,4-dimethylbenzyl)urea: mp 101-102 °C (recrystallized from isopropyl ether). Yield 80.2%.
Elemental analysis (for $C_{25}H_{24}F_2N_2O$)
Calculated: C, 73.87; H, 5.95; N, 6.89;
Found: C, 74.82; H, 5.94; N, 6.91.

Example 16

N-(2,4-Difluorophenyl)-N′-(2-indanyl)-N′-(2,5-dimethylbenzyl)urea: mp 127-128 °C (recrystallized from isopropyl ether). Yield 91.5%.
Elemental analysis (for $C_{25}H_{24}F_2N_2O$)
Calculated: C, 73.87; H, 5.95; N, 6.89;
Found: C, 73.97; H, 6.00; N, 6.81.

Example 17

N-(2,4-Difluorophenyl)-N′-(2-indanyl)-N′-(2,4,6-trimethylbenzyl)urea: mp 133-134 °C (recrystallized from isopropyl ether). Yield 80.5%.
Elemental analysis (for $C_{26}H_{26}F_2N_2O$)
Calculated: C, 74.27; H, 6.23; N, 6.66;
Found: C, 74.22; H, 6.17; N, 6.55.

Example 18

N-(2,4-Difluorophenyl)-N′-(2-indanyl)-N′-[1-(4-isopropylphenyl)ethyl]urea: mp 145-146 °C recrystallized from ethanol). Yield 93.1%.
Elemental analysis (for $C_{27}H_{28}F_2N_2O$)
Calculated: C, 74.63; H, 6.49; N, 6.45;
Found: C, 74.71; H, 6.58; N, 6.38.

Example 19

N-(2,4-Difluorophenyl)-N′-(2-indanyl)-N′-(2-methoxybenzyl)urea: mp 106-107 °C (recrystallized from ethanol). Yield 95.1%.
Elemental analysis (for $C_{24}H_{22}F_2N_2O_2$)
Calculated: C, 70.58; H, 5.43; N, 6.86;
Found: C, 70.88; H, 5.40; N, 6.91.

Example 20

N-(2,4-Difluorophenyl)-N′-(2-indanyl)-N′-(2,4-dimethoxybenzyl)urea: mp 114-115 °C (recrystallized from ethanol). Yield 96.6%.
Elemental analysis (for $C_{25}H_{24}F_2N_2O_3$)
Calculated: C, 68.48; H, 5.52; N, 6.39;
Found: C, 68.34; H, 5.55; N, 6.31.

Example 21

N-(2,4-Difluorophenyl)-N′-(2-indanyl)-N′-(3,4-dimethoxybenzyl)urea: mp 147-148 °C (recrystallized from ethanol). Yield 92.4%.
Elemental analysis (for $C_{25}H_{24}F_2N_2O_3$)
Calculated: C, 68.48; H, 5.52; N, 6.39;
Found: C, 68.42; H, 5.51; N, 6.36.

Example 22

N-(2,4-Difluorophenyl)-N′-(2-indanyl)-N′-(3,4,5-trimethoxybenzyl)urea: mp 126-127 °C (recrystallized from ethanol). Yield 78.7%.
Elemental analysis (for $C_{26}H_{26}F_2N_2O_4$)
Calculated: C, 66.66; H, 5.59; N, 5.98;

Found: C, 66.63; H, 5.58; N, 5.91.

Example 23

N-(2,4-Difluorophenyl)-N′-(2-indanyl)-N′-(2-thienylmethyl)urea: mp 66-67° C (recrystallized from isopropyl ether). Yield 97.4%.
Elemental analysis (for $C_{21}H_{18}F_2N_2OS$)
Calculated: C, 65.61; H, 4.72; N, 7.29;
Found: C, 65.56; H, 4.71; N, 7.23.

Example 24

N-(2,4-Difluorophenyl)-N′-(2-indanyl)-N′-(2-pyridylmethyl)urea: mp 105-106° C (recrystallized from ethanol). Yield 96.5%.
Elemental analysis (for $C_{22}H_{19}F_2N_3O$) Calculated: C, 69.65; H, 5.05; N, 11.08;
Found: C, 69.32; H, 5.08; N, 10.95.

Example 25

N-(2,4-Difluorophenyl)-N′-(2-indanyl)-N′-(3-pyridylmethyl)urea: mp 130-131° C (recrystallized from ethanolether). Yield 90.8%.
Elemental analysis (for $C_{22}H_{19}F_2N_3O$)
Calculated: C, 69.65; H, 5.05; N, 11.08;
Found: C, 69.47; H, 5.17; N, 10.79.

Example 26

N-Cyclohexylmethyl-N′-(2,4-difluorophenyl)-N-(2-indanyl)urea: mp 124-125° C (recrystallized from isopropyl ether). Yield 85.7%.
Elemental analysis (for $C_{23}H_{26}F_2N_2O$)
Calculated: C, 71.85; H, 6.82; N, 7.29;
Found: C, 72.15; H, 6.86; N, 7.30.

Example 27

N-Benzyl-N′-(2,4-difluorophenyl)-N-(4,6-dimethyl-2-indanyl)urea: mp 81-82° C (recrystallized from isopropyl ether). Yield 88.0%.
Elemental analysis (for $C_{25}H_{24}F_2N_2O$)
Calculated: C, 73.87; H, 5.95; N, 6.89;
Found: C, 74.02; H, 5.99; N, 6.91.

Example 28

N-Benzyl-N′-(2,4-difluorophenyl)-N-(4,7-dimethoxy-2-indanyl)urea: mp 110-111° C (recrystallized from ethanol). Yield 87.4%.
Elemental analysis (for $C_{25}H_{24}F_2N_2O_3$)
Calculated: C, 68.48; H, 5.52; N, 6.38;
Found: C, 68.31; H, 5.37; N, 6.30.

Example 29

N-Benzyl-N'-(2,4-difluorophenyl)-N-(5,6-dimethoxy-2-indanyl)urea: mp 116-117 °C (recrystallized from ethanol). Yield 68.2%.
Elemental analysis (for $C_{25}H_{24}F_2N_2O_3$)
Calculated: C, 68.48; H, 5.52; N, 6.39;
Found: C, 68.21; H, 5.52; N, 6.43.

Example 30

N-Benzyl-N'-(2,4-difluorophenyl)-N-(4,5,6-trimethoxy-2-indanyl)urea; yield 74.4% (powder).
Elemental analysis (for $C_{26}H_{26}F_2N_2O_4$)
Calculated: C, 66.66; H, 5.59; N, 5.98;
Found: C, 66.74; H, 5.61; N, 5.94.

Example 31

N-Benzyl-N'-(2,4-difluorophenyl)-N-(4,7-dimethoxy-5,6-dimethyl-2-indanyl)urea: mp 127-128 °C (recrystallized from ethanol). Yield 89.6%.
Elemental analysis (for $C_{27}H_{28}F_2N_2O_3$)
Calculated: C, 69.51; H, 6.05; N, 6.00;
Found: C, 69.35; H, 6.15; N, 5.89.

Example 32

N-(2,4-Difluorophenyl)-N'-(2-hydroxy-4-methoxybenzyl)-N'-(2-indanyl)urea: mp 165-166 °C (recrystallized from ethanol). Yield 87.8%.
Elemental analysis (for $C_{24}H_{22}F_2N_2O_3$)
Calculated: C, 67.92; H, 5.22; N, 6.60;
Found: C, 67.70; H, 5.20; N, 6.67.

Example 33

N-(2,4-Difluorophenyl)-N'-(4-hydroxy-3-methoxybenzyl)-N'-(2-indanyl)urea: mp 130-131 °C (recrystallized from isopropyl ether). Yield 76.5%.
Elemental analysis (for $C_{24}H_{22}F_2N_2O_3$)
Calculated: C, 67.92; H, 5.22; N, 6.60;
Found: C, 67.86; H, 5.21; N, 6.59.

Example 34

N-(2,4-Difluorophenyl)-N'-(4-hydroxy-3,5-dimethoxybenzyl)-N'-(2-indanyl)urea: mp 163-164 °C (recrystallized from isopropyl ether). Yield 70.9%.
Elemental analysis (for $C_{25}H_{24}F_2N_2O_4$)
Calculated: C, 66.07; H, 5.32; N, 6.16;
Found: C, 65.83; H, 5.35; N, 5.96.

Example 35

N-(2,4-Difluorophenyl)-N'-(2-indanyl)-N'-(2,5-dimethoxy-3,4-dimethylbenzyl)urea: mp 116-117 °C (recrystallized from ethanol). Yield 98.7%.
Elemental analysis (for $C_{27}H_{28}F_2N_2O_3$)
Calculated: C, 69.51; H, 6.05: N, 6.00:
Found: C, 69.34; H, 6.06; N, 5.97.

Example 36

N-(2,4-Difluorophenyl)-N′-(2-indanyl)-N′-(4-methoxy-2,5-dimethylbenzyl)urea: mp 140-141 °C (recrystallized from ethanol). Yield 96.9%.
Elemental analysis (for $C_{26}H_{26}F_2N_2O_2$)
Calculated: C, 71.54; H, 6.00; N, 6.42;
Found: C, 71.53; H, 6.05; N, 6.39.

Example 37

N-(2,4-Difluorophenyl)-N′-(4-hydroxy-2,5-dimethylbenzyl)-N′-(2-indanyl)urea: mp 199-200 °C (recrystallized from ethyl acetate). Yield 52.1%.
Elemental analysis (for $C_{25}H_{24}F_2N_2O_2$)
Calculated: C, 71.08; H, 5.73; N, 6.63;
Found: C, 71.25; H, 5.76; N, 6.59.

Example 38

N-(2,4-Difluorophenyl)-N′-(4-hydroxy-3,5-dimethyl benzyl)-N′-(2-indanyl)urea: mp 170-171 °C (recrystallized from ethyl acetate-hexane). Yield 29.1%.
Elemental analysis (for $C_{25}H_{24}F_2N_2O_2$)
Calculated: C, 71.08; H, 5.73; N, 6.63;
Found: C, 70.83; H, 5.62; N, 6.55.

Example 39

N-(2,4-Difluorophenyl)-N′-(4-hydroxy-2,3,5-trimethylbenzyl)-N′-(2-indanyl)urea: mp 184-185 °C (recrystallized from ethanol). Yield 80.3%.
Elemental analysis (for $C_{26}H_{26}F_2N_2O_2$)
Calculated: C, 71.54; H, 6.00; N, 6.42;
Found: C, 71.53; H, 6.05; N, 6.37.

Example 40

N-(2,4-Difluorophenyl)-N′-(4-hydroxy-3,5-diisopropylbenzyl)-N′-(2-indanyl)urea: mp 161-162 °C (recrystallized from ethanol). Yiled 75.5%.
Elemental analysis (for $C_{29}H_{32}F_2N_2O_2$)
Calculated: C, 72.78; H, 6.74; N, 5.85;
Found: C, 62.61; H, 6.79; N, 5.80.

Example 41

N-(5-tert-Butyl-4-hydroxy-2-methylbenzyl)-N′-(2,4-difluorophenyl)-N-(2-indanyl)urea: mp 117-119 °C (recrystallized from ethanol). Yield 82.3%.
Elemental analysis (for $C_{28}H_{30}F_2N_2O_2 \cdot 1/2C_2H_5OH$)
Calculated: C, 71.44; H, 6.82; N, 5.75;
Found: C, 71.74; H, 6.84; N, 5.69.

Example 42

N-(3,5-Di-tert-butyl-4-hydroxybenzyl)-N′-(2,4-difluorophenyl)-N-(2-indanyl)urea: mp 125-126 °C (recrystallized from hexane). Yield 70.0%.

Elemental analysis (for $C_{31}H_{36}F_2N_2O_2$)
Calculated: C, 73.49; H, 7.16; N, 5.53;
Found: C, 73.29; H, 7.17; N, 5.53.

Example 43

N-[1-(3,5-Di-tert-butyl-4-hydroxyphenyl)ethyl]-N′- (2,4-difluorophenyl)-N-(2-indanyl)urea: mp 183-185 °C (recrystallized from ethanol). Yield 67.6%.
Elemental analysis (for $C_{32}H_{38}F_2N_2O_2$)
Calculated: C, 73.82; H, 7.36; N, 5.38;
Found: C, 74.00; H, 7.42; N, 5.53.

Example 44

N-Benzyl-N′-(2,4-difluorophenyl)-N-(1,2,3,4-tetrahydro-2-naphthyl)urea: mp 103-104 °C (recrystallized from ethanol-hexane). Yield 91.6%.
Elemental analysis (for $C_{24}H_{22}F_2N_2O$)
Calculated: C, 73.45; H, 5.65; N, 7.14;
Found: C, 73.26; H, 5.57; N, 7.18.

Example 45

N-(2,4-Difluorophenyl)-N′-(4-hydroxy-5-isopropyl-2-methylbenzyl)-N′-(2-indanyl)urea: mp 198-199 °C (recrystallized from ethanol). Yield 96.7%.
Elemental analysis (for $C_{27}H_{28}F_2N_2O_2$)
Calculated: C, 71.98; H, 6.26; N, 6.22;
Found: C, 71.78; H, 6.27; N, 6.20.

Example 46

N-(2,4-Difluorophenyl)-N′-(4-hydroxy-2-isopropyl-5-methylbenzyl)-N′-(2-indanyl)urea: mp 196-197 °C (recrystallized from ethanol). Yield 99.6%.
Elemental analysis (for $C_{27}H_{28}F_2N_2O_2$)
Calculated: C, 71.98; H, 6.26; N, 6.22;
Found: C, 72.04; H, 6.21; N, 6.28.

Example 47

N-(5-tert-Butyl-4-hydroxy-2-methyl-3-propylbenzyl)-N′-(2,4-difluorophenyl)-N-(2-indanyl)urea: mp 157-158 °C (recrystallized from ethanol). Yield 99.4%.
Elemental analysis (for $C_{31}H_{36}F_2N_2O_2$)
Calculated: C, 73.49; H, 7.16; N, 5.53;
Found: C, 73.63; H, 7.10; N, 5.53.

Example 48

N-(2,4-Difluorophenyl)-N′-(2-indanyl)-N′-(4- methylthiobenzyl)urea: mp 102-103 °C (recrystallized from ethanol). Yield 92.0%.
Elemental analysis (for $C_{24}H_{22}F_2N_2OS$)
Calculated: C, 67.90; H, 5.22; N, 6.60;
Found: C, 68.15; H, 5.19; N, 6.63.

Example 49

N-Benzyl-N′-(2,4-difluorophenyl)-N-(6,7,8,9-tetrahydro-5H-6-benzocycloheptenyl)urea: mp 78-80 °C (recrystallized from isopropyl ether-hexane). Yield 79.3%.
Elemental analysis (for $C_{25}H_{24}F_2N_2O$)
Calculated: C, 73.87; H, 5.95; N, 6.89;
Found: C, 74.06; H, 5.95; N, 6.92.

Example 50

N-(5-tert-Butyl-4-hydroxy-2-methylbenzyl)-N′-(2,4-difluorophenyl)-N-(6,7,8,9-tetrahydro-5H-6-benzocycloheptenyl)urea: mp 188-189 °C (recrystallized from acetone-isopropyl ether). Yield 94.5%.
Elemental analysis (for $C_{30}H_{34}F_2N_2O_2$)
Calculated: C, 73.15; H, 6.96; N, 5.69;
Found: C, 73.38; H, 7.13; N, 5.64.

Example 51

N-(3,5-di-tert-Butyl-4-hydroxybenzyl)-N′-(2,4-difluorophenyl)-N-(6,7,8,9-tetrahydro-5H-6-benzocycloheptenyl)urea: mp 175-176 °C (recrystallized from ethanol). Yield 91.6%.
Elemental analysis (for $C_{33}H_{40}F_2N_2O_2$)
Calculated: C, 74.13; H, 7.54; N, 5.24;
Found: C, 74.13; H, 7.61; N, 5.11.

Example 52

To a solution of 2,4,6-trimethylbenzoic acid (328 mg) and diphenylphosphoryl azide (DPPA, 660 mg) in benzene (8.0 ml) was added dropwise triethylamine (0.28 ml) and the mixture was stirred at room temperature for 20 minutes and heated under reflux for 30 minutes to give 2,4,6-trimethylphenyl isocyanate. The reaction mixture was cooled to room temperature, followed by addition of 2-(3,5-di-tert-butyl-4-hydroxybenzylamino)indan hydrochloride (580 m) and, then, triethylamine (0.21 ml). The mixture was stirred at room temperature for 4 hours, washed successively with water, a saturated aqueous solution of $NaHCO_3$ and water and dried over anhydrous $MgSO_4$. The solvent was distilled off and the residue was crystallized from hexane to give N-(3,5-di-tert-butyl-4-hydroxybenzyl)-N-(2-indanyl)-N′-(2,4,6-trimethylphenyl(urea (702 mg, 91.3%). Recrystallization from isopropyl ether gave colorless needles (496 mg, 64.6%).
mp 104-106 °C.
Elemental analysis (for $C_{34}H_{44}N_2O_2$)
Calculated: C, 79.65; H, 8.65; N, 5.46;
Found: C, 79.36; H, 8.73; N, 5.31.

Example 53

N-(3,5-Di-tert-butyl-4-hydroxybenzyl)-N-(2-indanyl)-N′-(2,4-dimethylphenyl)urea was obtained in the same manner as Example 52. mp 173-174 °C (recrystallized from isopropyl alcohol). Yield 61.7%.
Elemental analysis (for $C_{33}H_{42}N_2O_2$)
Calculated: C, 79.48; H, 8.49; N, 5.62;
Found: C, 79.33; H, 8.55; N, 5.47.

Example 54

N-(2,4-Difluorophenyl)-N′-(4-hydroxy-3,5-dimethoxybenzyl)-N′-(6,7,8,9-tetrahydro-5H-6-benzocycloheptenyl)urea was obtained as colorless prisms in the same manner as Example 1. Yield 84.4 %.

mp 187-188 °C (recrystallized from ethanol)
Elemental analysis (for $C_{27}H_{28}F_2N_2O_4$)
Calculated: C, 67.21; H, 5.85; N, 5.81;
Found: C, 66.93; H, 5.80; N, 5.74.

Example 55

N-(2,4-Difluorophenyl(-N′-(6,7,8,9-tetrahydro-5H-6-benzocycloheptenyl)-N′-(4-trifluoromethylbenzyl)urea was obtained as a colorless powder in the same manner as Example 1. Yield 75.5%.
Elemental analysis (for $C_{26}H_{23}F_5N_2O$)
Calculated: C, 65.82; H, 4.89; N, 5.90;
Found: C, 66.05; H, 4.93; N, 5.83.

Example 56

N-(2,4-Difluorophenyl)-N′-(6,7,8,9-tetrahydro-5H-6-benzocycloheptenyl)-N′-(2,5-dimethylbenzyl)urea was obtained as a colorless powder in the same manner as Example 1. Yield 83.4%.
Elemental analysis (for $C_{27}H_{28}F_2N_2O$)
Calculated: C, 74.63; H, 6.49; N, 6.45;
Found: C, 74.72; H, 6.48; N, 6.52.

Example 57

N-Benzyl-N′-(2,4-difluorophenyl)-N-(1-indanyl)urea was obtained as colorless prisms in the same manner as Example 1. Yield 80.5%.
mp 101-102 °C (recrystallized from ethanol-hexane).
Elemental analysis (for $C_{23}H_{20}F_2N_2O$)
Calculated: C, 73.00; H, 5.33; N, 7.40;
Found: C, 72.78; H, 5.35; N, 7.34.

Example 58

N-Benzyl-N′-(2,4-difluorophenyl)-N-(1,2,3,4-tetrahydro-1-naphthyl)urea was obtained as colorless prisms in the same manner as Example 1. Yield 83.1%.
mp 149-150 °C (recrystallized from ethanol).
Elemental analysis (for $C_{24}H_{22}F_2N_2O$)
Calculated: C, 73.45; H, 5.65; N, 7.14;
Found: C, 73.44; H, 5.79; N, 7.19.

Example 59

N-Benzyl-N′-(2,4-difluorophenyl)-N-(6,7,8,9-tetrahydro-5H-5-benzocycloheptenyl)urea was obtained as colorless needles in the same manner as Example 1. Yield 64.2%.
mp 120-121 °C (recrystallized from ethanol-hexane).
Elemental analysis (for $C_{25}H_{24}F_2N_2O$)
Calculated: C, 73.87; H, 5.95; N, 6.89;
Found: C, 73.93; H, 6.00; N, 6.80.

Example 60

N-(2,4-Difluorophenyl)-N′-(4-hydroxy-3,5-dimethoxybenzyl)-N′-(1-indanyl)urea was obtained as colorless needles in the same manner as Example 1. Yield 70.3%.

mp 133-134° C (recrystallized from ethanol).
Elemental analysis (for $C_{25}H_{24}F_2N_2O_4$)
Calculated: C, 66.07; H, 5.32; N, 6.16;
Found: C, 66.02; H, 5.32; N, 6.17.

Example 61

N-(2,4-Difluorophenyl)-N′-(4-hydroxy-3,5-dimethoxybenzyl)-N′-(1,2,3,4-tetrahydro-1-naphthyl)urea was obtained as colorless prisms in the same manner as Example 1. Yield 48.3%.
mp 96-98° C (recrystallized from ether).
Elemental analysis (for $C_{26}H_{26}F_2N_2O_4$)
Calculated: C, 66.66; H, 5.59; N, 5.98;
Found: C, 66.43; H, 5.69; N, 5.88.

Example 62

N-(2,4-Difluorophenyl)-N′-(6,7,8,9-tetrahydro-5H-5-benzocycloheptenyl)-N′-(4-hydroxy-3,5-dimethoxybenzyl)urea was obtained as colorless needles in the same manner as Example 1. Yield 61.5%.
mp 91-93° C (recrystallized from ethanol-hexane).
Elemental analysis (for $C_{27}H_{28}F_2N_2O_4$)
Calculated: C, 67.21; H, 5.85; N, 5.81;
Found: C, 67.14; H, 5.79; N, 5.81.

Example 63

N-(4-Chromanyl)-N′-(2,4-difluorophenyl)-N-(4-hydroxy-3,5-dimethoxybenzyl)urea was obtained as colorless needles in the same manner as Example 1. Yield 41.7%.
mp 122-123° C (recrystallized from ethanol-hexane).
Elemental analysis (for $C_{25}H_{25}F_2N_2O_5$)
Calculated: C, 63.82; H, 5.14; N, 5.95;
Found: C, 63.75; H, 5.11; N, 5.95.

Example 64

N-Benzyl-N-(7-chloro-4-chromanyl)-N′-(2,4-difluorophenyl)urea was obtained as colorless prisms in the same manner as Example 1. Yield 64.7%.
mp 148-149° C (recrystallized from ethanol). Elemental analysis (for $C_{23}H_{19}C\ell F_2N_2O_2$)
Calculated: C, 64.41; H, 4.47; N, 6.53;
Found: C, 64.39; H, 4.49; N, 6.47.

Example 65

N-Benzyl-N′-(2,4-difluorophenyl)-N-(1-thiochroman-4-yl)urea was obtained as colorless needles in the same manner as Example 1. Yield 80.0%.
mp 143-144° C (recrystallized from ethanol).
Elemental analysis (for $C_{23}H_{20}F_2N_2OS$)
Calculated: C, 67.30; H, 4.91; N, 6.82;
Found: C, 67.43; H, 4.96; N, 6.74.

Reference Example 1

To a mixture of 2-indanone (1.32 g), 2-chlorobenzylamine (1.42 g), acetic acid (1.8 mℓ) and methanol

(20 mℓ) was added dropwise a solution of sodium cyanoborohydride (0.6 g) in methanol (3.0 mℓ), with constant stirring. The mixture was further stirred at room temperature for 3 hours and 6 N-HCℓ (4.0 mℓ) was then added. The mixture was further stirred for 30 minutes, at the end of which time it was made alkaline with 6 N-NaOH, diluted with water and extracted with ethyl acetate. The extract was washed with water and dried over anhydrous $MgSO_4$. The solvent was then distilled off and the residue was dissolved in ethanol (5.0 mℓ) followed by addition of 5 N-HCℓ-ethanol (4.0 mℓ), whereupon 2-(2-chlorobenzylamino)-indan hydrochloride was obtained as crystals (2.0 g, 68.0%). Recrystallization from ethanol gave colorless platelets (1.37 g, 46.6%).

mp 230-232 °C.

Elemental analysis (for $C_{16}H_{16}C\ell N \cdot HC\ell$)

Calculated: C, 65.32; H, 5.82; N, 4.76;

Found: C, 65.32; H, 5.83; N, 4.68.

The compounds of Reference Example 2 to 28 were produced in the same manner as above.

Reference Example 2

2-Benzylaminoindan hydrochloride: mp 230-235 °C.

Reference Example 3

2-Phenylaminoindan p-toluenesulfonate: mp 224-225 °C.

Reference Example 4

2-(2-Phenylethylamino)indan hydrochloride: mp 255-256 °C.

Reference Example 5

2-(4-Chlorobenzylamino)indan hydrochloride: mp 256-258 °C.

Reference Example 6

2-(1-Phenylethylamino)indan hydrochloride: mp 240-241 °C.

Reference Example 7

2-(1-Phenylpropylamino)indan hydrochloride: mp 218-220 °C.

Reference Example 8

2-(2-Methyl-1-phenylpropylamino)indan: mp 73-74 °C.

Reference Example 9

2-Diphenylmethylaminoindan: mp 74-75 °C.

Reference Example 10

2-(2-Methylbenzylamino)indan hydrochloride: mp 250-252 °C.

Reference Example 11

2-(3-Methylbenzylamino)indan hydrochloride: mp 259-260 °C.

Reference Example 12

2-(4-Methylbenzylamino)indan hydrochloride: mp 250-251 °C.

Reference Example 13

2-(4-Isopropylbenzylamino)indan hydrochloride: mp 235-238 °C.

Reference Example 14

2-(4-tert-Butylbenzylamino)indan hydrochloride: mp 276-278 °C.

Reference Example 15

2-(2,4-Dimethylbenzylamino)indan hydrochloride: mp 253-254 °C.

Reference Example 16

2-(2,5-Dimethylbenzylamino)indan hydrochloride: mp 265-266 °C.

Reference Example 17

2-[1-(4-Isopropylphenyl)ethylamino]indan hydrochloride: mp 263-265 °C.

Reference Example 18

2-(2-Methoxybenzylamino)indan hydrochloride: mp 192-193 °C.

Reference Example 19

2-(2,4-Methoxybenzylamino)indan hydrochloride: mp 215-216 °C.

Reference Example 20

2-(3,4-Dimethoxybenzylamino)indan hydrochloride: mp 231-232 °C.

Reference Example 21

2-(3,4,5-Trimethoxybenzylamino)indan hydrochloride: mp 235-236 °C.

Reference Example 22

2-(2-Thienylmethylamino)indan hydrochloride: mp 214-215 °C.

Reference Example 23

2-(2-Pyridylmethylamino)indan dihydrochloride: mp 202-205 °C.

Reference Example 24

2-(3-Pyridylmethylamino)indan dihydrochloride: mp 225-227 °C.

Reference Example 25

2-Cyclohexylmethylaminoindan hydrochloride: mp 251-253 °C.

Reference Example 26

2-(3,5-Di-tert-butyl-4-hydroxybenzylamino)indan hydrochloride: mp 228-231 °C.

Reference Example 27

2-[1-(3,5-Di-tert-butyl-4-hydroxyphenyl)ethylamino]indan hydrochloride: oily substance.

Reference Example 28

2-(2-Benzylamino)-1,2,3,4-tetrahydronaphthalene hydrochloride: mp 242-245 °C.

Reference Example 29

A solution of sodium cyanoborohydride (0.6 g) in methanol (3 ml) was added dropwise to a mixture of 2-aminoindan (1.33 g), 2,4,6-trimethylbenzaldehyde (1.48 g), acetic acid (1.8 ml) and methanol (15 ml) and the resulting mixture was stirred at room temperature for 3 hours, followed by addition of 6N-HCl (6.0 ml). The mixture was further stirred for 30 minutes, then made alkaline with 6N-NaOH and, after addition of water, extracted with ethyl acetate. The extract was washed with water and dried over anhydrous $MgSO_4$. The solvent was distilled off and the residue was dissolved in ethanol (3 ml). Addition of 5N ethanolic HCl to the solution gave 2-(2,4,6-trimethylbenzylamino)indan hydrochloride as crystals (2.2 g, 73.1%). Recrystallization from ethanol gave colorless platelets (2.01 g, 66.8%).
mp 282-283 °C.
Elemental analysis (for $C_{19}H_{23}N \cdot HCl$)
Calculated: C, 75.60; H, 8.01; N, 4.64;
Found: C, 75.64; H, 8.14; N, 4.63.

The compounds of Reference Examples 30 to 46 were produced in the same manner as Reference Example 29.

Reference Example 30

2-Benzylamino-5,6-dimethoxyindan hydrochloride: mp 273-275 °C (decomposition).

Reference Example 31

2-(4-Hydroxy-3,5-dimethoxybenzylamino)indan hydrochloride: 199-200 °C.

Reference Example 32

2-(4-Hydroxy-3,5-dimethylbenzylamino)indan: mp 171-172 °C (monohydrade).

Reference Example 33

2-(2,5-Dimethoxy-3,4-dimethylbenzylamino)indan hydrochloride: mp 212-213 °C.

Reference Example 34

2-(4-Hydroxy-2,5-dimethylbenzylamino)indan hydrochloride: mp 267-268 °C.

Reference Example 35

2-(4-Methoxy-2,5-dimethylbenzylamino)indan hydrochloride: mp 253-254 °C.

Reference Example 36

2-(4-Hydroxy-3,5-diisopropylbenzylamino)indan hydrochloride: mp 180-182 °C.

Reference Example 37

2-(4-Hydroxy-2,3,5-trimethylbenzylamino)indan: mp 146-147 °C.

Reference Example 38

2-(4-Hydroxy-3-methoxybenzylamino)indan: mp 118-119 °C.

Reference Example 39

2-(2-Hydroxy-4-methoxybenzylamino)indan: mp 105-106 °C.

Reference Example 40

2-(5-tert-Butyl-4-hydroxy-2-methylbenzylamino)indan: mp 169-170 °C.

Reference Example 41

2-(4-Hydroxy-5-isopropyl-2-methylbenzylamino)indan: mp 150-151 °C.

Reference Example 42

2-(4-Hydroxy-2-isopropyl-5-methylbenzylamino)indan hydrochloride: mp 223-225 °C.

Reference Example 43

2-(5-tert-Butyl-4-hydroxy-2-methyl-3-propylbenzylamino)indan: mp 110-111 °C.

Reference Example 44

2-(4-Methylthiobenzylamino)indan hydrochloride: mp 245-248 °C.

Reference Example 45

6-Benzylamino-6,7,8,9-tetrahydro-5H-benzocycloheptene hydrochloride: mp 220-221 °C

Reference Example 46

2-(5-tert-Butyl-4-hydroxy-2-methylbenzylamino)-6,7,8,9-tetrahydro-5H-benzocycloheptene: mp 152-153 °C.

Reference Example 47

Benzoyl chloride (0.62 ml) was added dropwise to a mixture of 2-amino-4,6-dimethylindan hydrochloride (1.0 g), potassium carbonate (0.84 g), ethyl acetate (10 ml) and water (10 ml) with stirring and ice cooling. The resultant mixture was stirred with ice cooling for 1 hour. The organic layer was then separated, washed with water and dried over anhydrous $MgSO_4$ and the solvent was distilled off to give 2-benzoylamino-4,6-dimethylindan as crystals (1.2 g, 89.6%).
Recrystallization from isopropyl alcohol gave colorless needles (1.1 g, 82.1%).
mp 119-120 °C.

A mixture of the above crystals (1.0 g), lithium aluminum hydride (0.21 g) and dry tetrahydrofuran (10 ml) was heated under reflux for 9 hours. Water (1.0 ml) was added dropwise to the reaction mixture with ice cooling and the precipitate was filtered off. The solvent was distilled off from the filtrate and 5N ethanolic HCl was added to the residue, whereby 2-benzylamino-4,6-dimethylindan hydrochloride was obtained as crystals (0.4 g, 37.0%). Recrystallization from ethanol gave colorless needles (0.3 g, 27.8%). mp 267-268 °C.
Elemental analysis (for $C_{18}H_{21}N \cdot HCl$)
Calculated: C, 75.11; H, 7.70; N, 4.87;
Found: C, 74.85; H, 7.73; N, 4.66.

The compounds of Reference Examples 48 to 50 were produced in the same manner as Reference Example 47.

Reference Example 48

2-Benzoylamino-4,7-dimethoxyindan: mp 216-217 °C.
2-Benzylamino-4,7-dimethoxyindan hydrochloride: mp 237-239 °C.

Reference Example 49

2-Benzoylamino-4,5,6-trimethoxyindan: mp 125-126 °C.
2-Benzylamino-4,5,6-trimethoxyindan hydrochloride: mp 206-207 °C.

Reference Example 50

2-Benzoylamino-4,7-dimethoxy-5,6-dimethylindan: mp 173-174 °C.
2-Benzylamino-4,7-dimethoxy-5,6-dimethylindan hydrochloride: mp 230-231 °C.

Reference Example 51

To a solution of 4,7-dimethoxy-1-indanone (5.8 g) in ethyl acetate (60 ml) were added isoamyl nitrite (4.85 ml) and 4N-HCl-ethyl acetate (6 ml) dropwise and the mixture was stirred at room temperature for 5 hours to give 2-oxyimino-4,7-dimethoxy-1-indanone as crystals (5.3 g; 79.9%). Recrystallization from methanol-chloroform gave yellow needles. mp 156-157 °C.

The above crystals (5 g) were mixed with 80% acetic acid (100 ml) and concentrated sulfuric acid (5.0 ml) and the resulting mixture was subjected to hydrogenation in the presence of 5% palladium on charcoal (2.5 g) under atmospheric pressure and at room temperature for 3 days. The catalyst was then filtered off and after the solvent was distilled off, the residue was diluted with water, neutralized with potassium carbonate, and extracted with chloroform. The extract was dried over anhydrous $MgSO_4$ and the solvent was distilled off. The residue was dissolved in ethyl acetate (30 ml) followed by addition of 5N HCl-ethanol to give 2-amino-4,7-dimethoxyindan hydrochloride as crystals (3.8 g; 73.2%).
Recrystallization from ethanol gave colorless needles. mp 253-255 °C.
Elemental analysis (for $C_{11}H_{15}NO_2 \cdot HC\ell$)
Calculated: C, 57.52; H, 7.02; N, 6.10;
Found: C, 57.49; H, 7.04; N, 6.08.

The compounds of Reference Examples 52 to 54 were produced in the same manner as Reference Example 51.

Reference Example 52

2-Oxyimino-1-benzosuberone: mp 137-138 °C.
6-Amino-6,7,8,9-tetrahydro-5H-benzocycloheptene hydrochloride: mp 224-226 °C.

Reference Example 53

2-Oxyimino-4,6-dimethyl-1-indanone: mp 237-239 °C.
2-Amino-4,6-dimethylindan hydrochloride: mp 280-283 °C (decomposition).

Reference Example 54

2-Oxyimino-4,7-dimethoxy-5,6-dimethyl-1-indanone: mp 244-245 °C.
2-Amino-4,7-dimethoxy-5,6-dimethylindan hydrochloride: mp 292-296 °C (decomposition).

The compounds of Reference Examples 55 to 61 were produced in the same manner as Reference Example 29.

Reference Example 55

6,7,8,9-Tetrahydro-6-(4-trifluoromethylbenzylamino)-5H-benzocycloheptene hydrochloride: mp 241-243 °C.

Reference Example 56

6-(4-Hydroxy-3,5-dimethoxybenzylamino)-6,7,8,9- tetrahydro-5H-benzocycloheptene: mp 140-141 °C.

Reference Example 57

6-(2,5-Dimethylbenzylamino)-6,7,8,9-tetrahydro-5H-benzocycloheptene hydrochloride: mp 238-240 °C.

Reference Example 58

5-(4-Hydroxy-3,5-dimethoxybenzylamino)-6,7,8,9-tetrahydro-5H-benzocycloheptene p-toluenesulfonate:

mp 98-103° C.

Reference Example 59

1-(4-Hydroxy-3,5-dimethoxybenzylamino)indan oxalate: mp 204-205° C.

Reference Example 60

1-(4-Hydroxy-3,5-dimethoxybenzylamino)-1,2,3,4-tetrahydronaphthalene p-toluenesulfonate: mp 198-200° C.

Reference Example 61

4-(4-Hydroxy-3,5-dimethoxybenzylamino)chroman oxalate: mp 189-190° C.

Reference Example 62

A mixture of 1-indanone (2.64 g), benzylamine (2.36 g), p-toluenesulfonic acid hydrate (0.6 g) and benzene (60 ml) was refluxed using a Dean-Stark apparatus for 7 hours. After cooling, the insolubles were filtered off and the filtrate was distilled to remove the solvent. The residue was dissolved in methanol (40 ml) and $NaBH_4$ (1.0 g) was added gradually with ice-cooling. The mixture was stirred under ice-cooling for 1 hour and at room temperature for 15 hours. It was then diluted with water and extracted with ethyl acetate. The extract was washed with water and dried ($MgSO_4$) and the solvent was distilled off. The residue was treated with 5N HCl-AcOEt to give 1-benzylaminoindan hydrochloride as crystals.
Recrystallization from ethanol-ether gave colorless prisms (2.48 g; 47.7%).
mp 183-184° C.
Elemental analysis (for $C_{16}H_{17}N \cdot HCl$)
Calculated: C, 73.98; H, 6.98; N, 5.39;
Found: C, 74.02; H, 7.04; N, 5.04.

The compounds of Reference Examples 63 to 66 were produced in the same manner as Example 62.

Reference Example 63

1-Benzylamino-1,2,3,4-tetrahydronaphthalene hydrochloride: mp 175-179° C.

Reference Example 64

5-Benzylamino-6,7,8,9-tetrahydro-5H-benzocycloheptene hydrochloride: mp 199-210° C.

Reference Example 65

4-Benzylamino-1-thiochroman hydrochloride: mp 143-144° C.

Reference Example 66

4-Benzylamino-7-chlorochroman hydrochloride: mp 193-196° C.

**Claims**

1. A benzocycloalkane derivative of the formula (I):

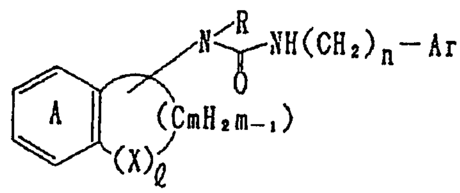

(I)

wherein R is a hydrogen atom or a hydrocarbon residue, which may optionally have one or more substituents, Ar is an aromatic group, X is an oxygen or sulfur atom, $\ell$ is 0 or 1, m is 3 to 6 and n is 0 to 2, wherein the ring A and the group Ar each may optionally have one or more substituents, with the proviso that compounds are excluded, wherein Ar is

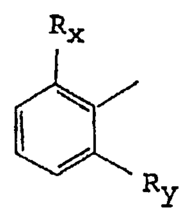

whereby $R_x$ and $R_y$ are independently selected from alkyl or alkoxy of from one to seven carbon atoms, or its pharmaceutically acceptable salt.

2. A benzocycloalkane derivative of claim 1 in which the derivative of the formula (I) is a compound of the formula (I'):

(I')

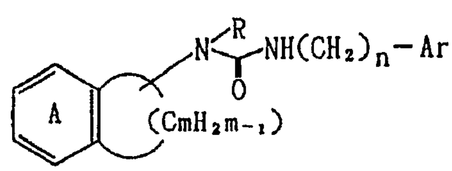

wherein R is a hydrogen atom or a hydrocarbon residue, which may optionally have one or more substituents, Ar is an aromatic group, m is 3 to 6 and n is 0 to 2 and wherein the ring A and the group Ar each may optionally have one or more substituents, with the proviso that compounds are excluded, wherein Ar is

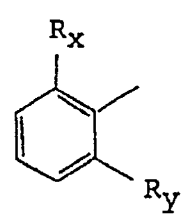

whereby $R_x$ and $R_y$ are independently selected from alkyl or alkoxy of from one to seven carbon atoms.

3. A benzocycloalkane derivative of claim 1, wherein R is a hydrogen atom, an alkyl group having 1-8 carbon atoms, an aryl group having 6-10 carbon atoms, or an aralkyl group having 7-16 carbon atoms.

4. A benzocycloalkane derivative of claim 1, wherein the hydrocarbon residue represented by R has 1 to 5 substituents, which may be the same or different, selected from the group consisting of a halogen atom, an optionally halogenated alkoxy or alkylthio having 1-6 carbon atoms, nitro, a carboxy or $C_{1-6}$ alkyl-esterified carboxy, hydroxy, an acyl group having 1-3 carbon atoms and a 5- or 6-membered aromatic heterocyclic group.

5. A benzocycloalkane derivative of claim 1, wherein the hydrocarbon residue represented by R is a benzyl group which may be substituted with an alkyl having 1-4 carbon atoms, an alkoxy having 1-4 carbon

atoms, hydroxy or a halogen atom.

6. A benzocycloalkane derivative of claim 1, wherein the group Ar is an aryl group having 6-10 carbon atoms or a 5- or 6-membered aromatic heterocyclic group and n is 0.

7. A benzocycloalkane derivative of claim 1, wherein each of the ring A and the group Ar has one to five substituents selected from the group consisting of a halogen atom, an optionally halogenated alkoxy group having 1-6 carbon atoms, an optionally halogenated alkyl having 1-6 carbon atoms, an optionally halogenated alkylthio group having 1-6 carbon atoms, nitro group, a carboxy or $C_{1-6}$ alkyl-esterified carboxyl group, hydroxy group, an acyl group having 1-3 carbon atoms and a 5- or 6-membered aromatic heterocyclic group.

8. A benzocycloalkane derivative of claim 1, wherein the group Ar is a mono- or di-halogen-substituted aryl group having 6-10 carbon atoms.

9. A benzocycloalkane derivative of claim 8, wherein the halogen-substituted aryl group is a mono- or di-fluorine-substituted phenyl group.

10. A benzocycloalkane derivative of claim 1 in which the benzocycloalkane derivative of the formula (I) is a compound of the formula ($I^a$):

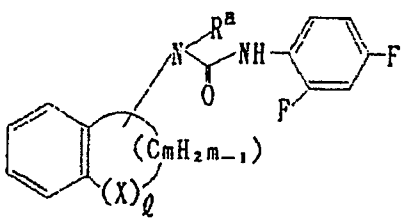

($I^a$)

wherein $R^a$ is a benzyl group, which may optionally have one or more substituents, X is an oxygen or sulfur atom, $\ell$ is 0 or 1 and m is 3 to 6.

11. A benzocycloalkane derivative of claim 1 in which the benzocycloalkane derivative of the formula (I) is a compound of the formula ($I^b$):

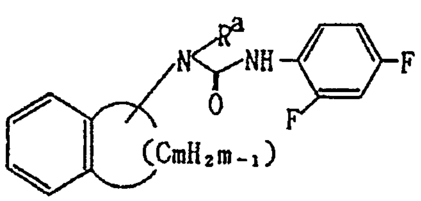

($I^b$)

wherein $R^a$ is a benzyl group, which may optionally have one or more substitutents, and m is 3 to 6.

12. A benzocycloalkane derivative of claim 1 in which the benzocycloalkane derivative of the formula (I) is
N-(4-isopropylbenzyl)-N′-(2,4-difluorophenyl)-N-(2-indanyl)urea,
N-(2,4-difluorophenyl)-N′-(2-indanyl)-N′-(2,4-dimethylbenzyl)urea,
N-(2,4-difluorophenyl)-N′-(2-indanyl)-N′-(2,5-dimethylbenzyl urea,
N-(2,4-difluorophenyl)-N′-(4-hydroxy-2,5-dimethylbenzyl)-N′-(2-indanyl)urea,
N-(2,4-difluorophenyl)-N′-(4-hydroxy-2,3,5-trimethylbenzyl)-N′-(2-indanyl)urea,
N-(5-tert-butyl-4-hydroxy-2-methylbenzyl)-N,-(2,4-difluorophenyl)-N-(2-indanyl)urea,
N-(2,4-difluorophenyl)-N′-(4-hydroxy-5-isopropyl-2-methylbenzyl)-N′-(2-indanyl)urea,
N-benzyl-N′-(2,4-difluorophenyl)-N-(6,7,8,9-tetrahydro-5H-6-benzocycloheptenyl)urea,
N-(5-tert-butyl-4-hydroxy-2-methylbenzyl)-N′-(2,4-difluorophenyl)-N-(6,7,8,9-tetrahydro-5H-6-benzocycloheptenyl)urea, or
N-(2,4-difluorophenyl)-N′-(6,7,8,9-tetrahydro-5H-6-benzocycloheptenyl)-N′-(2,5-dimethylbenzyl) urea.

13. A composition for inhibiting acyl-CoA: cholesterol-acyl-transferase which comprises a benzocycloalkane derivative of any one of claims 1 to 12 and a pharmaceutically acceptable carrier or diluent.

14. A method of producing a benzocycloalkane derivative of the formula (I):

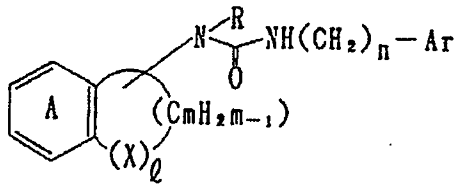
(I)

wherein R is a hydrogen atom or a hydrocarbon residue, which may optionally have one or more substituents, Ar is an aromatic group, X is an oxygen or sulfur atom, $\ell$ is 0 or 1, m is 3 to 6 and n is 0 to 2 and wherein the ring A and the group Ar each may optionally have one or more substituents, with the proviso that compounds are excluded, wherein Ar is

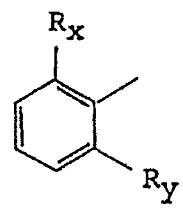

whereby $R_x$ and $R_y$ are independently selected from alkyl or alkoxy of from one to seven carbon atoms, or its pharmaceutically acceptable salt, which comprises reacting a compound of the formula (II):

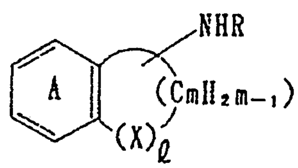

wherein the symbols are as defined above, or a salt thereof with a compound of the formula (III):
$Ar-(CH_2)_nNCO$ (III)
wherein the symbols are as defined above.

15. A method of claim 14, wherein the benzocycloalkane derivative (I) is a compound of the formula (I′):

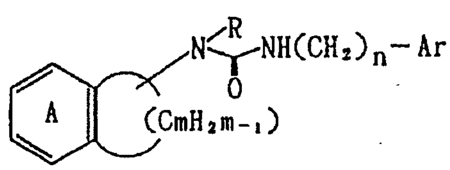
(I′)

wherein the symbols are as defined in claim 14, which comprises reacting a compound of the formula (II′):

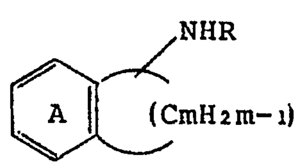
(II′)

wherein the symbols are as defined in claim 14, or a salt thereof, with a compound of the formula (III):
$Ar-(CH_2)_nNCO$ (III)
wherein the symbols are as defined in claim 14.

16. Use of a benzocycloalkane derivative of any one of claims 1 to 12 for the production of a medicine for use in the prevention or treatment of disorders or diseases associated with acyl-CoA : cholesterol acyltransferase.

European Patent Office

# EUROPEAN SEARCH REPORT

Application number

EP 90109570.3

## DOCUMENTS CONSIDERED TO BE RELEVANT

| Category | Citation of document with indication, where appropriate, of relevant passages | Relevant to claim | CLASSIFICATION OF THE APPLICATION (Int Cl.5) |
|---|---|---|---|
| A | DE - A1 - 2 718 947 (SCHERICO) * Claims 1,3,5 * | 1,13, 14 | C 07 C 275/30<br>C 07 C 323/44<br>C 07 C 275/26<br>C 07 D 333/12<br>C 07 D 213/36<br>C 07 D 311/20<br>C 07 D 335/06<br>A 61 K 31/17 |
| A | DE - A1 - 2 623 417 (AMERICAN CYANAMID) * Claims 1,4 * | 1,14 | |
| A | GB - A - 1 479 544 (AMERICAN CYANAMID) * Claims 1,13 * | 1,14 | |
| D,A | US - A - 4 623 662 (VERN G. DE VRIES) * Abstract; column 1, lines 11-24 * | 1,13 | |

TECHNICAL FIELDS SEARCHED (Int Cl.5)

C 07 C 275/00
C 07 C 323/00
C 07 D 333/00
C 07 D 213/00
C 07 D 311/00
C 07 D 335/00

The present search report has been drawn up for all claims

| Place of search | Date of completion of the search | Examiner |
|---|---|---|
| VIENNA | 28-08-1990 | REIF |

### CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

EPO Form 1503 03 82